# FASCICULE DE BREVET EUROPEEN

(11) **EP 2 687 221 B1**
(45) Date de publication et mention de la délivrance du brevet: **13.04.2016**
(21) Numéro de dépôt: 13174825.3
(22) Date de dépôt: 03.07.2013
(51) Int. Cl.: A61K 36/42, A61K 36/49, A61P 1/12, A61P 31/04

(54) **Composition à base de Nekka-Rich pour la prévention de pathologies intestinales**
Zusammensetzung auf der Basis von Nekka-Rich zur Prävention von Darmkrankheiten
Nekka-Rich composition for the prevention of intestinal diseases

(30) Priorité: 17.07.2012 FR 1256878
(43) Date de publication de la demande: 22.01.2014
(73) Titulaire: KAZTRADE Corporation, 276-0046 (JP)
(72) Inventeur: Besnier, Patrick, 88300 Certilleux (FR)
(74) Mandataire: Schmidt, Martin Peter

(56) Documents cités:
- EP-A2- 0 481 396
- WATARAI S ET AL: "Feeding Activated Charcoal from Bark Containing Wood Vinegar Liquid (Nekka-Rich) Is Effective as Treatment for Cryptosporidiosis in Calves", JOURNAL OF DAIRY SCIENCE, AMERICAN DAIRY SCIENCE ASSOCIATION, US, vol. 91, no. 4, 1 avril 2008 (2008-04-01), pages 1458-1463, XP026956498, ISSN: 0022-0302 [extrait le 2008-04-01]
- VORAVUTHIKUNCHAI SUPAYANG PIYAWAN ET AL: "Antibacterial activities of semipurified fractions of Quercus infectoria against enterohemorrhagic Escherichia coli O157 : H7 and its verocytotoxin production", JOURNAL OF FOOD PROTECTION, vol. 71, no. 6, juin 2008 (2008-06), pages 1223-1227, XP009167701, ISSN: 0362-028X

## Description

### Domaine technique de l'invention

La présente invention concerne le traitement préventif de pathologies intestinales, en particulier le traitement préventif de la turista humaine et des diarrhées colibacillaires à souche entérotoxinogène (ECET) chez l'homme et chez les animaux mammifères, en particulier chez le veau, le chevreau, l'agneau ou encore les animaux domestiques.

### Etat de la technique

La turista (ou « tourista »), communément appelée diarrhée du voyageur, est le plus souvent d'origine bactérienne (environ 80 % des cas). l'Escherichia coli entérotoxinogène (ou ECET) est l'agent pathogène responsable de cette pathologie. Chaque année, l'Escherichia coli entérotoxinogène (ou ECET) provoque entre 280 et 400 millions de cas de diarrhée chez les enfants de moins de 5 ans, 100 millions entre 5 et 14 ans et 400 millions après 15 ans, pour la plupart dans les pays en voie de développement.

La turista touche près de 20 % à 50 % des voyageurs en séjour de courte durée, et cela le plus souvent dans les pays en voie de développement. Cette infection intervient généralement une semaine après l'arrivée du voyageur et peut durer plusieurs jours, et donc devenir suffisamment gênant pour troubler son séjour. L'ingestion d'aliments ou d'eau contaminés est la principale source d'infection de la turista. De plus, dans 10% des cas, l'infection évoluera vers une pathologie chronique gênante, le syndrome du colon irritable.

Actuellement, il n'existe pas de vaccin universel qui permettrait de prévenir la turista. En revanche, certains médicaments, à base de sous-salicylate de bismuth, ont été utilisés à titre préventif et ont démontré une certaine efficacité vis-à-vis de la turista. En revanche, les effets secondaires de ces médicaments ne sont pas négligeables : apparition d'anxiété, faiblesse musculaire, constipation, spasmes musculaires. On trouve également sur le marché de nombreux produits contenant des pro-biotiques, i.e. des micro-organismes vivants ajoutés comme complément alimentaire dans certains produits tels que des yaourts, dont l'efficacité sur la turista n'a pas été démontrée.

L'Escherichia coli entérotoxinogène (ECET) est également impliquée dans les diarrhées néonatales des veaux, des agneaux et des chevreaux. Cet agent pathogène entraîne alors une utilisation d'antibiotiques de manière plus ou moins raisonnée. Par conséquent, les souches ECET présentent une résistance de plus en plus importante face aux antibiotiques. Ainsi, les souches ECET isolées des diarrhées néonatales présentent plus de 85 % de résistance à des antibiotiques tels que l'amoxicilline. Dès 1995, une sous-population (5 %) des souches d'Escherichia coli ECET issues de diarrhées du veau présentent une résistance au florfénicol, un puissant antibiotique bactériostatique. En 2009, environ 20 % des souches ECET sont devenues résistantes à cet antibiotique, dont certaines hébergent même simultanément le gène responsable (floR) et certains gènes de résistance aux céphalosporines de troisième génération (blaCMY-2).

Le but de la présente invention est de proposer une composition permettant de prévenir de manière efficace les infections intestinales d'origine bactérienne liées à la présence de germe de type Escherichia coli entérotoxinogène (ECET). Plus particulièrement, le but de la présente invention est de proposer une composition à utiliser à titre préventif pour éviter une éventuelle infection intestinale de type « turista » pour l'homme.

Un autre but de l'invention est de proposer une composition efficace pour le traitement préventif d'infections intestinales provoquées par l'Escherichia coli entérotoxinogène (ECET) chez les animaux tels que les veaux, les chevreaux, les agneaux, ou les animaux domestiques carnivores tels que les chiens et les chats, et donc permettant de diminuer l'incidence et la gravité des diarrhées ainsi que l'utilisation systématique d'antibiotiques.

En effet, l'Escherichia coli, appelé aussi E.coli ou colibacille, est une bactérie de la flore intestinale que l'on retrouve dans la plupart des espèces animales. Cette bactérie existe sous différentes formes dont certaines peuvent être pathogènes et provoquer des infections intestinales, et notamment d'induire une diarrhée. On distingue quatre formes différentes d'Escherichia coli :
- l'E.coli entéropathogène, responsable de gastro-entérites infantiles sécrétant une toxine voisine de la toxine des shigelles (connue aussi sous le terme anglais « shigella like ») ;
- l'E.coli entérotoxinogène (ECET) responsable de diarrhées infantiles dans les pays chauds à hygiène déficiente et de la diarrhée des voyageurs (turista) ;
- l'E.coli entéro-invasif, envahissant les cellules épithéliales du gros intestin et pouvant créer des ulcérations. Il secrète une toxine voisine de la toxine des shigelles ;
- l'E.coli entérohémorragique, pouvant produire une diarrhée hémorragique et pouvant s'aggraver d'un syndrome hémolytique et urémique. Il produit de puissantes cytotoxines.

La présente invention se limite au groupe d'E.coli entérotoxinogène. Ces souches produisent deux types de toxines : les toxines thermolabile et thermostable, responsables de l'apparition de la diarrhée en induisant une hypersécrétion de fluides dans la lumière intestinale. Lors d'une contamination, l'ECET colonise l'intestin grêle et déclenche la sécrétion de cytokines jouant un rôle important dans le mécanisme régulateur de la réponse immunitaire et inflammatoire.

### Objets de l'invention

La présente invention a pour objet une composition à base d'extraits d'écorces de bois de chêne (*Castanopsis cuspidata* et/ou *Quercus acuta*) comprenant à titre de principe actif le Nekka-Rich pour son utilisation pour le traitement préventif et/ou curatif de pathologies intestinales provoquées par la bactérie Escherichia coli entérotoxinogène (ECET) chez l'homme ou chez les animaux mammifères.

La composition est avantageusement en association ou en mélange avec au moins un excipient ou véhicule inerte et non toxique.

Dans un mode de réalisation particulier, la composition comprend en outre au moins un additif, de préférence sélectionné dans le groupe formé par :
- les vitamines A, E, D, C synthétiques ou naturelles ;
- les oligo-éléments, et notamment le fer, l'iode, le sélénium, le zinc, le cuivre, le cobalt, le manganèse, sous leur forme minérale ou organique ;
- des extraits de *macleaya cordata* ;
- les argiles, et notamment la montmorillonite, la bentonite, la smectite ;
- les pro-biotiques tels que les lactobacillus ou les saccharomyces ;
- les anti-diarrhéique ;
- les antispasmodiques ;
- les antiseptiques intestinaux, et notamment des nitrofuranes ou des quinoléines.

Avantageusement, la composition se présente sous forme solide, notamment sous la forme d'un comprimé compressé, d'une gélule, d'une poudre orale, d'une poudre en sachet ; ou sous forme liquide, notamment sous la forme d'un gel, d'une émulsion, d'une mousse, d'une pâte ou d'une suspension.

Selon un mode de réalisation particulier, la composition sous forme solide destinée à l'homme comprend entre 10 g à 100 g de Nekka-Rich pour 100 g de préparation, de préférence 30 g à 100 g de Nekka-Rich pour 100 g de préparation, et encore plus préférentiellement 60 g à 100 g de Nekka-Rich pour 100 g de préparation.

Dans un autre mode de réalisation, la composition sous forme solide destinée aux animaux comprend entre 10 g à 100 g de Nekka-Rich pour 100 g de préparation, de préférence 30 g à 100 g Nekka-Rich pour 100 g de préparation, et encore plus préférentiellement 45 g à 100 g de Nekka-Rich pour 100 g de préparation.

Selon un mode de réalisation particulier, la composition sous forme liquide destinée à l'homme comprend entre 20 g à 600 g de Nekka-Rich pour 1 litre de préparation, de préférence 100 g à 450 g de Nekka-Rich pour 1 litre de préparation, et encore plus préférentiellement 250 g à 350 g de Nekka-Rich pour 1 litre de préparation.

Dans un autre mode de réalisation, la composition sous forme liquide destinée aux animaux comprend entre 20 g à 600 g de Nekka-Rich pour 1 litre de préparation, de préférence 100 g à 450 g Nekka-Rich pour 1 litre de préparation, et encore plus préférentiellement 250 g à 350 g de Nekka-Rich pour 1 litre de préparation.

L'invention a aussi pour objet la composition selon l'invention pour utilisation par voie orale chez l'homme, à raison de deux prises quotidiennes comprises entre 0,8 g et 1,2 g de Nekka-Rich par 15 kg de poids vif, et comprises entre 2,2 g et 2,7 g de Nekka-Rich pour un enfant de plus de 30 kg.

L'invention a également pour objet la composition selon l'invention pour utilisation par voie orale chez le jeune mammifère, à raison de deux prises quotidiennes, l'une le matin et l'autre le soir, et notamment :
(i) comprises entre 1,0 et 1,5 g chacune de Nekka-Rich chez l'agneau ou le chevreau de 10 kg ou moins de 10 kg ;
(ii) comprises entre 1,8 et 2,2 g chacune de Nekka-Rich chez l'agneau ou le chevreau de plus de 10 kg ;
(iii) comprises entre 4 et 6 g chacune de Nekka-Rich pour un veau de 40 kg ou de moins de 40 kg ;
(iv) comprises entre 7 et 8 g chacune de Nekka-Rich pour un veau entre 40 kg et 60 kg ou de 60 kg ;
(v) comprises entre 9 et 11 g chacune de Nekka-Rich pour un veau de plus de 60 kg ;
(vi) comprises entre 0,2 et 1 g chacune de Nekka-Rich pour un carnivore domestique de 5 kg ou moins de 5 kg ;
(vii) comprises entre 1,5 et 2 g chacune de Nekka-Rich pour un carnivore domestique entre 5 kg et 15 kg ou de 15 kg ;
(viii) comprises entre 3 et 4 g chacune de Nekka-Rich pour un carnivore domestique de entre 15 kg et 30 kg ou de 30 kg ;
(ix) comprises entre 4,5 et 6 g chacune de Nekka-Rich pour un carnivore domestique de plus de 30 kg.

Enfin, un autre objet de l'invention est la composition selon l'invention pour utilisation en tant que complément alimentaire pour l'homme ou en tant qu'aliment complémentaire pour les animaux.

### Présentation détaillée des figures

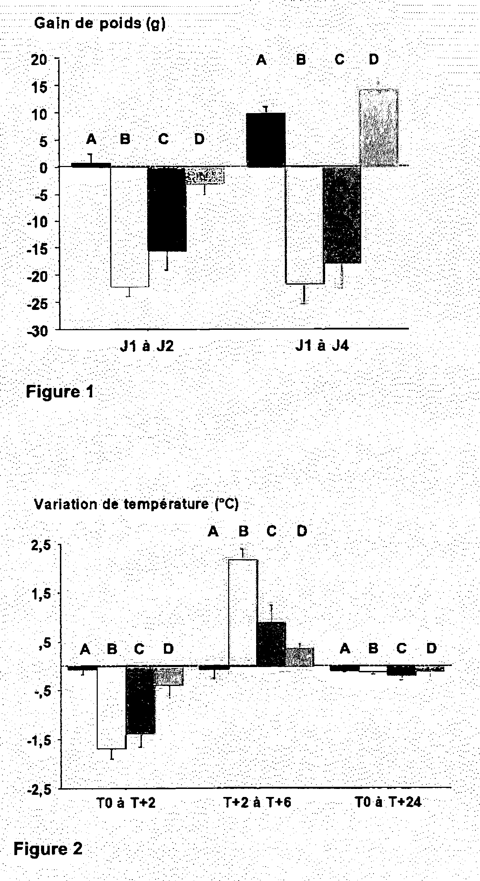

La figure 1 montre l'évolution des gains de poids moyens (exprimé en grammes) des rats des 4 groupes (Témoin/Véhicule = barre A ; Turista/Véhicule = barre B ; Turista/NK200 = barre C ; Turista/NK400 = barre D) de traitement entre la date J1 (i.e. juste avant de l'induction de la turista et le traitement unique par voie orale) et J2 (i.e. 24 h après l'induction de la turista et le traitement unique par voie orale) et entre J1 et J4 (i.e. 72 h après l'induction de la turista et le traitement unique par voie orale).
La figure 2 montre les variations de températures corporelles moyennes des rats des 4 groupes de traitement (Témoin/Véhicule = barre A ; Turista/Véhicule = barre B ; Turista/NK200 = barre C ; Turista/NK400 = barre D) entre T0 et T+2 correspondant à l'intervalle entre la température corporelle basale moyenne avant induction de la turista et la température corporelle moyenne la plus basse observée au cours de l'expérimentation 2 heures après induction de la turista, entre T+2 et T+6 correspondant à l'intervalle entre les températures corporelles moyennes les plus basse et élevée observées au cours de l'expérimentation 6 heures après induction de la turista et entre T0 et T+24 correspondant à l'intervalle entre la température corporelle basale moyenne avant induction de la turista et la température corporelle moyenne 24 heures après induction de la turista.

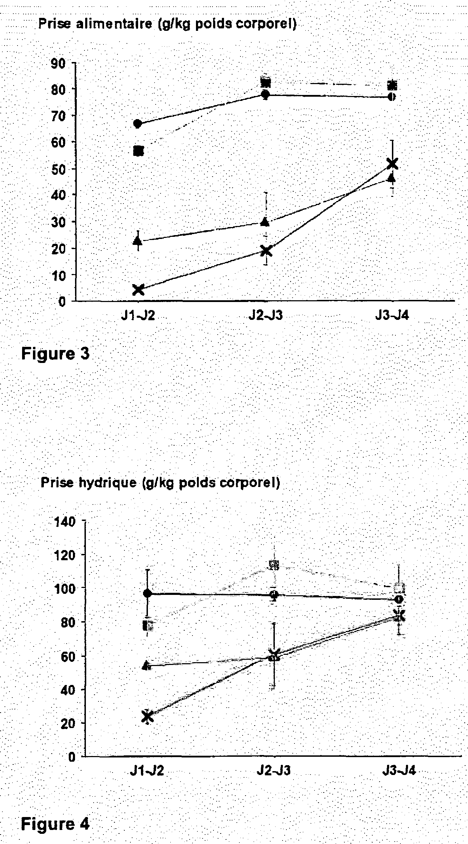

La figure 3 représente l'évolution de la prise alimentaire (exprimée en g/kg poids corporel) des rats des 4 groupes de traitement entre la date J1 (i.e. juste avant de l'induction de la turista et le traitement unique par voire orale) et J2 (i.e. 24 h après l'induction de la turista et le traitement unique par voire orale) ; entre J2 et J3 (i.e. 48 h après l'induction de la turista et le traitement unique par voire orale) et entre J3 et J4 (i.e. 72 h après l'induction de la turista et le traitement unique par voire orale).
   Ronds noirs : rats du groupe Témoin/Véhicule ;
   Croix : rats du groupe Turista/Véhicule ;
   Triangles : rats du groupe Turista/NK200 ;
   Carrés : rats du groupe Turista/NK400.
La figure 4 représente l'évolution de la prise hydrique (exprimée en g/kg poids corporel) des rats des 4 groupes de traitement entre la date J1 (i.e. juste avant de l'induction de la turista et le traitement unique par voire orale) et J2 (i.e. 24 h après l'induction de la turista et le traitement unique par voire orale) ; entre J2 et J3 (i.e. 48 h après l'induction de la turista et le traitement unique par voire orale) et entre J3 et J4 (i.e. 72 h après l'induction de la turista et le traitement unique par voire orale). Les points expérimentaux représentent les mêmes groupes de rats que dans la figure 3.

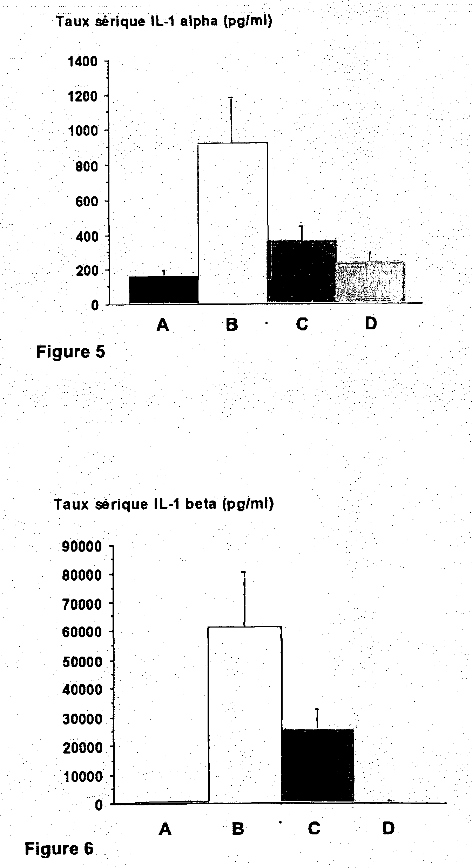

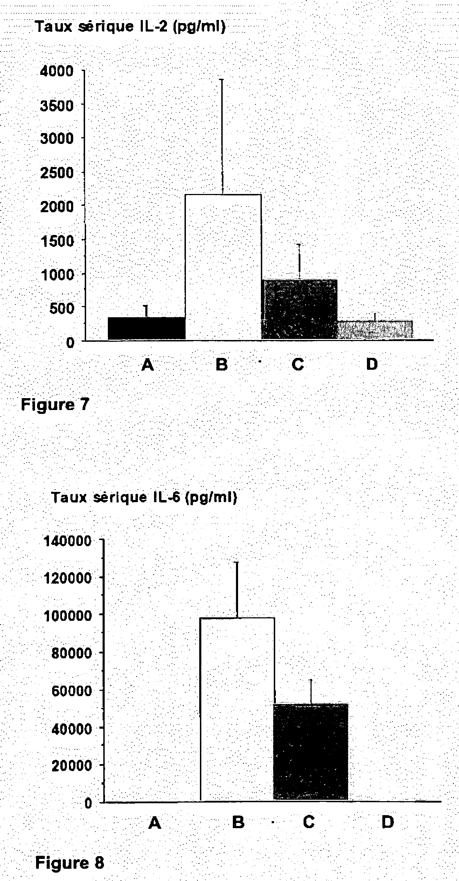

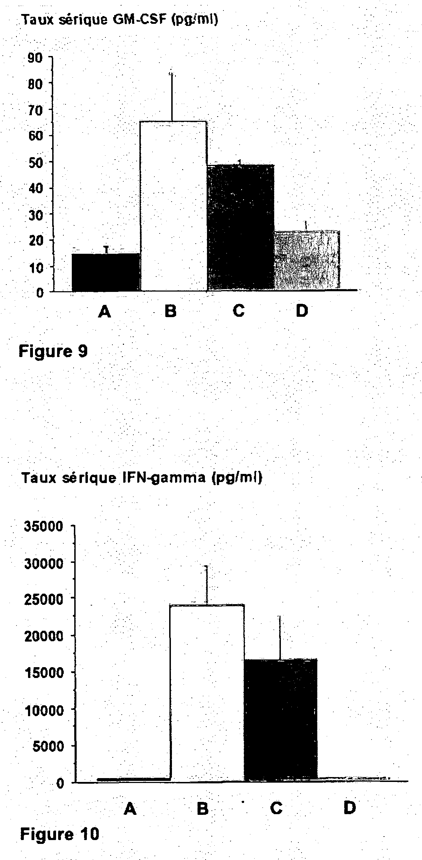

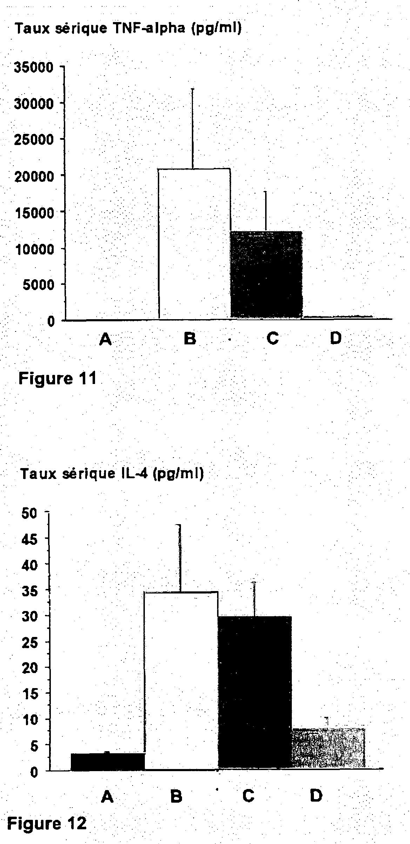

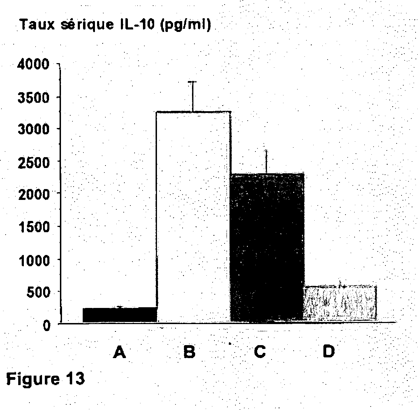

Dans les figures 5 à 13, on indique les taux sériques (exprimés en pg/ml) de différents protéines de quatre groupes de rats ayant reçus chacun un type de traitement différent : Témoin/Véhicule = barre A ; Turista/Véhicule = barre B ; Turista/NK200 = barre C ; Turista/NK400 = barre D. Ces taux sériques ont été mesurés 3 heures après l'introduction de la turista.

| | | | |
|---|---|---|---|
| Figure 5 : IL-1α. | Figure 6 : IL-1β. | Figure 7 : IL-2. | Figure 8 : IL-6. |
| Figure 9 : GM-CSF. | Figure 10 : IFN-γ. | Figure 11 : TNF-α. | |
| Figure 12 : IL-4. | Figure 13 : IL-10. | | |

### Description détaillée de l'invention

Les inventeurs ont découvert que des compositions à base de Nekka-Rich sont utiles pour la prévention de certaines pathologies intestinales et notamment de la turista.

Le Nekka-Rich est un charbon de bois actif, provenant de bois ou d'écorces de bois de chêne bien spécifique contenant du « vinaigre de bois ». Le procédé d'obtention du Nekka-Rich consiste essentiellement à brûler des écorces de bois de chêne (*Castanopsis cuspidata* et/ou *Quercus acuta*) en milieu anaérobie afin d'obtenir, d'une part, du charbon de bois, et, d'autre part, des fumées résultant de la décomposition de l'eau et des composés du bois contenus dans les cellules de bois, lesdites fumées étant refroidies pour obtenir par condensation un liquide qui est ensuite purifié afin de récupérer le vinaigre de bois. Le vinaigre de bois est ensuite déposé sur le charbon actif qui résulte dudit charbon de bois.

Ce produit est connu et s'avère efficace pour le traitement de la cryptosporidiose sur les animaux, en particulier les veaux, les agneaux ou les chevreaux (cf. *"*Feeding activated charcoal from bark containing wood vinegar liquid (nekka-rich) is effective as treatment for cryptosporidiosis in calves", Journal of Dairy Science 2008, 91(4), p. 1458-63; *"*Control of cryptosporidiosis in neonatal goat kids : Efficacy of a product containing activated charcoal and wood vinegar liquid (Obionekk®) in field conditions", Veterinary Parasitology, Volume 180, Issues 3-4, p. 354-357). Ce produit est également efficace pour le traitement de la salmonelle sur les animaux, en particulier sur les gallinacés (cf. « Eliminating the carriage of Salmonella enterica serovar enteritidis in domestic fowls by feeding activated charcoal from bark containing wood vinegar liquid (Nekka-Rich*) »).*

Dans le cadre de la présente invention, le rapport massique entre le vinaigre de bois et le charbon de bois compris dans le Nekka-Rich tel que défini ici peut varier entre 5 % et 20% massiques, c'est à dire que 20 kg de Nekka-Rich peuvent comprendre entre 1 et 4 kg de vinaigre de bois.

Afin de déterminer l'efficacité du Nekka-Rich en traitement préventif des pathologies intestinales, aussi bien chez l'être humain que chez les animaux, et afin de déterminer quantitativement les doses de Nekka-Rich à utiliser pour prévenir de telles pathologies, une étude a été réalisée sur des rats mâles Wistar Han (Centre d'élevage Harlan, Horst, Hollande) soumis a une turista induite par une administration orale de bactéries de type Escherichia Coli (souche ECET).

Cette étude a été effectuée sur une population de 24 rats mâles Wistar Han d'un poids compris entre 250 et 275 g. Les animaux ont été pesés puis répartis en 4 groupes distincts (n = 6) en fonction des traitements effectués :
- Premier groupe (appelé « groupe Témoin/Véhicule ») : pas de turista induite et traitement oral avec le véhicule.
- Deuxième groupe (appelé « groupe Turista/Véhicule ») : turista induite et traitement oral avec le véhicule.
- Troisième groupe (appelé « groupe Turista/NK200 ») : turista induite et traitement oral avec le Nekka-Rich à la dose de 200 mg/kg.
- Quatrième groupe (appelé « Turista/NK400 ») : turista induite et traitement oral avec le Nekka-Rich à la dose de 400 mg/kg.

Pour la réalisation de cette étude :
- Des bactéries Escherichia coli de la souche B (Escherichia Coli ATCC 11303 cells, EC11303 Sigma) de la société SIGMA-ALDRICH ont été utilisées pour induire la turista. Ces bactéries, se présentant sous la forme de lyophilisat, ont été conservées à une température de -20°C avant utilisation.
- Le Nekka-Rich sous la forme de préparation prête à l'emploi (importé du Japon par la société Obione) a été conservé à température ambiante et à l'abri de la lumière, de l'humidité et de la chaleur avant son utilisation.
- Les bactéries Escherichia coli ont été préparées en solution dans un sérum physiologique (NaCl, 0,9%) puis ont été maintenues au bain-marie à 37°C pendant 1 heure avant d'être administrées par voie orale aux animaux.
- De l'eau de source a été utilisée comme véhicule pour le traitement des animaux des groupes n°1 et n°2 et également pour diluer et obtenir les solutions de Nekka-Rich aux concentrations à tester avant d'être administrées par voir orale aux animaux des groupes n°3 et n°4.
- Des volumes de la préparation prête à l'emploi de Nekka-Rich ont été mesurés et placés dans des flacons et l'eau de source a été ajoutée à ces volumes mesurés afin de préparer des solutions de Nekka-Rich aux concentrations de 71,43 mg/ml et 142,86 mg/ml. Les solutions de Nekka-Rich préparées ont ensuite été placées sous agitation magnétique pendant 5 minutes.

**Tableau 1 : Traitement des rats**

| | Nombre de rats | Induction de la turista | Traitement par voie orale | Dose [mg/kg] |
|---|---|---|---|---|
| Groupe | | Produit administré | Produit administré | |
| Témoin + Véhicule | 6 | NaCl | Eau de Source | - |
| Turista + Véhicule | 6 | E.Coli | Eau de Source | - |
| Turista + Nekka-Rich 200 | 6 | E.Coli | Nekka-Rich | 200 |
| Turista + Nekka-Rich 400 | 6 | E.Coli | Nekka-Rich | 400 |

La turista a été induite chez les rats par une administration unique par voie orale de 20 ml/kg d'une solution de bactéries E. Coli de souche B préparée dans du sérum physiologique (NaCl, 0,9%) à la concentration de 1,5 mg/ml.

Les solutions de Nekka-Rich préparées aux concentrations de 71,43 mg/ml et 142,86 mg/ml ont été administrées par voie orale aux doses de 200 et 400 mg/kg respectivement 30 minutes après l'induction de la turista avec un volume d'administration de 10 ml/kg.

Le véhicule a été administré dans les mêmes conditions, 30 minutes après l'induction de la turista avec un volume d'administration de 10 ml/kg.

### Suivi des animaux

Les rats ont été pesés avant l'induction de la turista puis ont été pesés quotidiennement pendant 3 jours après induction. La viabilité et le comportement des rats ont été analysés tout au long de l'étude. Les paramètres pris en compte lors du suivi des animaux ont été :
- Le gain de poids moyen ;
- la variation de la température corporelle moyenne ;
- la prise alimentaire et la prise hydrique ;
- le taux de cytokines pro- et anti-inflammatoires ;
- la consistance des fèces.

Afin d'étudier la variation de la température corporelle moyenne des animaux, la température corporelle des animaux a été mesurée juste avant l'induction de la turista (2 points basaux) puis toutes les heures pendant 8 heures le jour de l'induction de cette pathologie. Le lendemain, 24 heures après l'induction de la turista, la température corporelle des animaux a été à nouveau mesurée.

En ce qui concerne le suivi des prises alimentaire et hydrique des animaux , un relevé des prises alimentaire et hydrique a été effectué quotidiennement après induction de la turista pour l'ensemble des cages de rats.

Un prélèvement de sang a été réalisé sur chaque rat entre 3 et 4 heures après l'induction de la turista. L'extrémité de la queue du rat a été coupée avec une lame de scalpel et environ 1 ml de sang a été recueilli dans un tube sec (Térumo, Leuven, Belgique) en massant légèrement la queue du rat. Les échantillons de sang récupérés ont été placés à +4°C pendant 20 à 30 minutes puis centrifugés à 1500g pendant 15 minutes. Les sérums ont été collectés dans des tubes Eppendorf, congelés à -20°C puis conservés à -80°C jusqu'au dosage des cytokines pro- et anti-inflammatoires.

En ce qui concerne le dosage des cytokines pro- et anti-inflammatoires, après décongélation de l'ensemble des échantillons de sérum, le dosage de 9 cytokines pro- et anti-inflammatoires, IL-1α, IL-1β, IL-2, IL-4, IL-6, IL-10, GM-CSF, IFN-γ et TNF-α a été réalisé simultanément à l'aide d'un kit Bio-Plex Rat Cytokine 9-plex A panel (Bio-Rad, Réf. 171-K11070, Marnes-la-Coquette, France) en utilisant la technique Bio-Plex. Les dosages ont effectués en double et en aveugle.

### Statistiques

On définit par « significatif » tout résultat statistique ne pouvant pas être obtenu par hasard. Pour cela, on utilise un seuil de signification (i.e. une probabilité notée P) de 0,05, ce qui signifie que le résultat observé a moins de 5 % de chances d'être obtenu par hasard. Par opposition, on défit par « non significatif » un résultat statistique qui a probablement (à plus de 5 % de chances) été obtenu par hasard.

Les paramètres pris en compte lors du suivi des animaux ont été analysés en fin d'expérimentation. Une analyse de la variance (ANOVA) a été réalisée en mode non paramétrique à l'aide du test de Kruskal-Wallis (KW). Le test KW est un test d'identité utilisé pour déterminer si « k » échantillons indépendants sont issus d'une même population.

En cas de significativité, i.e. pour une valeur de P < 0,05, le test de Kruskal-Wallis est suivi par le test U de Mann-Whitney afin de comparer les groupes traités avec le Nekka-Rich aux groupes témoins induits ou non. Le test Mann et Whitney U permet de comparer deux échantillons indépendants, de faibles effectifs, ne vérifiant pas la condition de "normalité". Les données de l'étude ont été représentées sous forme de moyennes ± ESM (erreur standard à la moyenne). Les traitements statistiques ont été réalisées à l'aide du logiciel Statview 5 (SAS, Institute Inc., USA).

### Gain de poids moyen

Le test de Kruskal-Wallis montre qu'il y a eu des différences significatives entre les gains de poids moyens des rats des 4 groupes de traitement entre J1 et J2 (P=0,002) (Tableau 2) et entre J1 et J4 (P=0,001) (Tableau 3).

Entre J1 et J2, les gains de poids moyens des rats des 2 groupes Turista/Véhicule et Turista/NK200 ont été significativement plus faibles que ceux des rats des 2 groupes Témoin/Véhicule (U=0,0 ; P=0,006 et U=2,0 ; P=0,010 respectivement) et Turista/NK400 (U=0,0 ; P=0,006 et U=4,0 ; P=0,024, respectivement). Une perte de poids significative a même été observée pour ces 2 groupes Turista/Véhicule et Turista/NK200. Aucune différence significative n'a été observée entre les gains de poids moyens des rats des groupes Témoin/Véhicule et Turista/NK400 et entre ceux des rats des groupes Turista/Véhicule et Turista/NK200 (Tableau 2).

Entre J1 et J4, les gains de poids moyens des rats des 2 groupes Turista/Véhicule et Turista/NK200 ont été significativement plus faibles que ceux des rats des 2 groupes Témoin/Véhicule (U=0,0 ; P=0,006 et U=0,5 ; P=0,005, respectivement) et Turista/NK400 (U=0,0 ; P=0,006 et U=0,0 ; P=0,004, respectivement). Une perte de poids significative a même été observée pour ces 2 groupes Turista/Véhicule et Turista/NK200. Le gain de poids moyen des rats du groupe Turista/NK400 a montré une tendance à être significativement plus élevé que celui des rats du groupe Témoin/Véhicule (U=6,5 et P=0,065). En revanche, aucune différence significative n'a été observée entre les gains de poids moyens des rats des groupes Turista/Véhicule et Turista/NK200 (Tableau 3).

**Tableau 2 :**

| Comparaison des gains moyens des rats (g) entre J1 et J2 (Moyenne ± ESM) | | | | |
|---|---|---|---|---|
| **Groupe** | **Témoin/Véhicule** | **Tourista/Véhicule** | **Tourista/NK200** | **Tourista/NK400** |
| **Moyenne ± ESM** | **+0.67 ± 1.56** | **-22.20 ± 1.66** | **-15.50 ± 3.62** | **-3.17 ± 1.96** |
| **Kruskal-Wallis H_{(ddl=3)}=15.41 P=0.015 U Mann-Whitney (*vs*. Témoin/Véhicule) Significativité** | | **U=0.0 *P*=*0.006*** | **U=2.0 *P*=*0.010*** | **U=8.5 *NS*** |
| **U Mann-Whitney (*vs*. Tourista/Véhicule) Significativité** | | | **U=7.0 *NS*** | **U=0.0 *P*=*0.006*** |
| **U Mann-Whitney (*vs*. Tourista/NK200) Significativité** | | | | **U=4.0 *P*=*0.024*** |

**Tableau 3 :**

| Comparaison des gains moyens des rats (g) entre J1 et J4 (Moyenne ± ESM) | | | | |
|---|---|---|---|---|
| **Groupe** | **Témoin/Véhicule** | **Tourista/Véhicule** | **Tourista/NK200** | **Tourista/NK400** |
| **Moyenne ± ESM** | **+9.97 ± 1.41** | **-21.60 ± 3.50** | **-17.83 ± 4.79** | **+13.83 ± 1.54** |
| **Kruskal-Wallis _{.} H_{(ddl=3)}=17.35; P=0.0001 U Mann-Whitney (*vs*. Témoin/Véhicule) significativité** | | **U=0.0 *P*=*0.006*** | **U=0.5 *P*=*0.005*** | **U=6.5 *T* (*P*=*0.065*)** |
| **U Mann-Whitney (*vs*. Touriste/Véhicule) significativité** | | | **U=12.5 *NS*** | **U=0.0 *P=0.006*** |
| **U Mann-Whitney (*vs.* Tourista/NK200) Significativité** | | | | **U=0.0 *P*=*0.004*** |

### Variation de température corporelle moyenne

Le test de Kruskal-Wallis montre qu'il y a eu des différences significatives entre les variations de températures corporelles moyennes des rats des 4 groupes de traitement entre T0 et T+2 (P=0,001) et entre T+2 et T+6 (P=0,001) (Tableaux 4 et 5), par contre aucune différence significative n'a été observée entre les variations de températures corporelle moyennes des rats des 4 groupes de traitement entre T0 et T+24 (P=0,33) (Tableau 6).

Entre T0 et T+2, les variations de températures corporelles moyennes des rats des 2 groupes Turista/Véhicule et Turista/NK200 ont été significativement plus élevées que celles des rats des 2 groupes Témoin/Véhicule (U=0,0 et P= 0.004 dans les 2 cas) et Turista/NK400 (U=1,5 ; P=0,008 et U=4,0 ; P=0,025 respectivement). Aucune différence significative n'a été observée entre les variations de températures corporelles moyennes des rats des groupes Témoin/Véhicule et Turista/NK400 et entre celles des rats des groupes Turista/Véhicule et Turista/NK200 (Tableau 4). Entre T+2 et T+6, les variations de températures corporelles moyennes des rats des 2 groupes Turista/Véhicule et Turista/NK200 ont été significativement plus élevées que celle des rats du groupe Témoin/Véhicule (U=0,0 ; P= 0,004 et U=5,5 ; P=0,045, respectivement) et la variation de température corporelle moyenne des rats du groupe Turista/Véhicule a été significativement plus élevée que celles des rats des groupes Turista/NK200 et Turista/NK400 (U=2,0 ; P=0,010 et U=0,0 ; P=0,004, respectivement). La variation de température corporelle moyenne des rats du groupe Turista/NK400 a montré une tendance à être significativement plus élevée que celles des rats du groupe Témoin/Véhicule (U=7,0 et P=0,072) et une tendance à être significativement plus faible que celle des rats du groupe Turista/NK200 (U=7,0 et P=0,076) (Tableau 5).

**Tableau 4 : Comparaison des variations de températures corporelles moyennes (°C) des rats entre T0 et T+2 (Moyenne ± ESM)**

| **Groupe** | **Témoin/Véhicule** | **Tourista/Véhicule** | **Tourista/NK200** | **Tourista/NK400** |
|---|---|---|---|---|
| **Moyenne ± ESM** | **-0.08 ± 0.11** | **-1.70 ± 0.20** | **-1.38 ± 0.28** | **-0.41 ± 0.24** |
| **Kruskal-Wallis H(_{ddl=3)} =15.53; P=0.001 U Mann-Whitney (*vs*. Témoin/Véhicule) Significativité** | | **U=0.0 *P*=*0.004*** | **U=0.0 *P=0.004*** | **U=12.0 *NS*** |
| **U Mann-Whitney (*vs*. Tourista/Véhicule) Significativité** | | | **U=13.0 *NS*** | **U=1.5 *P*=*0.008*** |
| **U Mann-Whitney (*vs*. Tourista/NK200 Significativité** | | | | **U=4.0 *P*=*0.025*** |

**Tableau 5 : Comparaison des variations de températures corporelles moyennes (°C) des rats entre T+2 et T+6 (Moyenne ± ESM)**

| **Groupe** | **Témoin/Véhicule** | **Tourista/Véhicule** | **Tourista/NK200** | **Tourista/NK400** |
|---|---|---|---|---|
| **Moyenne ± ESM** | **-0.08 ± 0-18** | **+2.17 ± 0.22** | **+0.87 ± 0.36** | **+0.33 ± 0.11** |
| **Kruskal-Wallis H_{(ddl=3)}=16.13; P=0.001 U Mann-Whitney (*vs*. Témoin/Véhicule) Significativité** | | **U=0.0 *P*=*0.004*** | **U=5.5 *P=0.045*** | **U=7.0 *T (P*=*0.072)*** |
| **U Mann-Whitney (*vs*. Tourista/Vèhicule) Significativité** | | | **U=2.0 *P*=*0.010*** | **U=0.0 *P*=*0.004*** |
| **U Mann-Whitney (vs. Tourista/NK200) Significativité** | | | | **U=7.0 *T (P*=*0.076)*** |

**Tableau 6 : Comparaison des variations de températures corporelles moyennes (°C) des rats entre T+6 et T+24 (Moyenne ± ESM)**

| **Groupe** | **Témoin/Véhicule** | **Tourista/Véhicule** | **Tourista/NK200** | **Tourista/NK400** |
|---|---|---|---|---|
| **Moyenne ± ESM** | **-0.09 ± 0.03** | **-0.12 ± 0-08** | **-021 ± 0.09** | **-0.12 ± 0.13** |

### Prise alimentaire

Le test de Kruskal-Wallis montre qu'il y a eu des différences significatives entre les prises alimentaires moyennes des animaux des 4 groupes de traitement entre J1 et J2 (P=0,016), entre J2 et J3 (P=0,035) et entre J3 et J4 (P=0,036) (cf. Tableaux 7 à 9).

En effet, entre J1 et J2, les prises alimentaires moyennes des rats des 3 groupes Turista/Véhicule, Turista/NK200 et Turista/NK400 ont été significativement plus faibles que celles des rats du groupe Témoin Véhicule (U=0,0 ; P=0,049 dans les 3 cas). Les prises alimentaires moyennes des rats des 2 groupes Turista/NK200 et Turista/NK400 ont été significativement plus élevées que celles des rats du groupe Turista/Véhicule (U=0,0 ; P=0,049 dans les deux cas). La prise alimentaire moyenne des rats du groupe Turista/NK400 a été significativement plus élevée que celle des rats du groupe Turista/NK200 (U=0,0 ; P=0,049) (cf. Tableau 7).

Entre J2 et J3 et entre J3 et J4, les prises alimentaires moyennes des rats des 2 groupes Turista/Véhicule et Turista/NK200 ont été significativement plus faibles que celles des rats des 2 groupes Témoin/Véhicule et Turista/NK400 (U=0,0 ; P=0,049 dans tous les cas). Aucune différence significative n'a été observée entre les prises alimentaires moyennes des rats des 2 groupes Témoin/Véhicule et Turista/NK400 et entre celles des rats des groupes Turista/Véhicule et Turista/NK200 (cf. Tableaux 8 et 9).

**Tableau 7 : Comparaison des prises alimentaires moyennes (g/kg poids corporel) des rats entre J1 et J2 (Moyenne ± ESM)**

| **Groupe** | **Témoin/Véhicule** | **Tourista/Véhicule** | **Tourista/NK200** | **Tourista/NK400** |
|---|---|---|---|---|
| **Moyenne** | **66.92** | **4.21** | **22.59** | **56.74** |
| **±** | **±** | **±** | **±** | **±** |
| **ESM** | **1.53** | **0.61** | **3.75** | **2.01** |
| **Kruskal-Wallis H_{(ddl-3)}=10.39; P=0.016 U Mann-Whitney (*vs*. Témoin/Véhicule) Significavité** | | **U=0.0 *P*=*0.049*** | **U=0.0 *P*=*0.049*** | **U=0.0 *P*=*0.049*** |
| **U Mann-Whitney (vs. Tourista/Véhicule) Signifïcativité** | | | **U=0.0 *P*=*0.049*** | **U=0.0 *P*=*0.049*** |
| **U Mann-Whitney (*vs*. Tourista/NK200) Significativité** | | | | **U=0.0 *P*=*0.049*** |

**Tableau 8 : Comparaison des prises alimentaires moyennes (g/kg poids corporel) des rats entre J2 et J3 (Moyenne ± ESM)**

| **Groupe** | **Témoin/Véhicule** | **Tourista/Véhicule** | **Tourista/NK200** | **Tourista/NK400** |
|---|---|---|---|---|
| **Moyenne ± ESM** | **77.92 ± 1.68** | **18.98 ± 5.52** | **29.42 ± 11.52** | **82.39 ± 3.12** |
| **Kruskal-Wallis H_{(ddl-3)} =8.64; P=0.035 U Mann-Whitney (*vs*. Témoin/Véhicule) Significativité** | | **U=0.0 *P*=*0.049*** | **U=0.0 *P*=*0.049*** | **U=2.0 *NS*** |
| **U Mann-Whitney (*vs*. Tourista/Véhicule) Significativité** | | | **U=4.0 *NS*** | **U=0.0 *P*=*0.049*** |
| **U Mann-Whitney (*vs*. Tourista/NK200) Significativité** | | | | **U=0.0 *P=0.049*** |

**Tableau 9 : Comparaison des prises alimentaires moyennes (g/kg poids corporel) des rats entre J3 et J4 (Moyenne ± ESM)**

| **Groupe** | **Témoin/Véhicule** | **Tourista/Véhicule** | **Tourista/NK200** | **Tourista/NK400** |
|---|---|---|---|---|
| **Moyenne ± ESM** | **76.71 ± 1.14** | **51.52 ± 9.03** | **45.87 ± 6.70** | **81.10 ± 6.36** |
| **Kruskal-Wallis H_{(ddl-3)} =8.54; P=0.036 U Mann-Whitney (vs. Témoin/Véhicule) Significativité** | | **U=0.0 *P*=*0.049*** | **U=0.0 *P=0.049*** | **U=3.0 *NS*** |
| **U Mann-Whitney (*vs*. Tourista/Véhicule) Significativité** | | | **U=3.0 *NS*** | **U=0.0 *P*=*0.049*** |
| **U Mann-Whitney (*vs*. Tourista/NK200) Significativité** | | | | **U=0.0 *P*=*0.049*** |

### Prise hydrique

Une analyse statistique de la prise hydrique moyenne quotidienne des animaux des 4 groupes de traitement au cours de l'expérimentation a été effectuée malgré le faible nombre de valeurs par point de mesure (n=3 par groupe).

Le test de Kruskal-Wallis montre qu'il y a eu une différence significative entre les prises hydriques moyennes des animaux des 4 groupes de traitement entre J1 et J2 (P=0,022) (Tableau 10). Par contre, aucune différence significative n'a été observée entre les prises hydriques moyennes des animaux des 4 groupes de traitement entre J2 et J3 (P=0,08) et entre J3 et J4 (P=0,54) (Tableaux 11 et 12).

Entre J1 et J2, les prises hydriques moyennes des rats des 2 groupes Turista/Véhicule et Turista/NK200 ont été significativement plus faibles que celles des rats des 2 groupes Témoin/Véhicule et Turista/NK400 (U=0,0 et P=0,049 dans tous les cas). La prise hydrique moyenne des rats du groupe Turista/Véhicule a été significativement plus faible que celle des rats du groupe Turista/NK200 (U=0,0 et P=0,049). En revanche, aucune différence significative n'a été observée entre les prises hydriques moyennes des rats des 2 groupes Témoin/Véhicule et Turista/NK400 (U=2,0 et P=0,28) (Tableau 11).

**Tableau 10 : Comparaison des prises hydriques moyennes (g/kg poids corporel) des rats entre J1 et J2 (Moyenne ± ESM)**

| **Groupe** | **Témoin/Véhicule** | **Tourista/Véhicule** | **Tourista/NK200** | **Tourista/NK400** |
|---|---|---|---|---|
| **Moyenne ± ESM** | **96.78 ± 14.01** | **23.95 ± 4.42** | **53.60 ± 1.71** | **77.66 ± 6.23** |
| **Kruskal-Wallis H_{(ddl-3)} =9.67; P=0.022 U Mann-Whitney (vs. Témoin/Véhicule) Significativité** | | **U=0.0 *P*=*0.049*** | **U=0.0 *P=0.049*** | **U=2.0 *NS*** |
| **U Mann-Whitney (*vs*. Tourista/Véhicule) Significativité** | | | **U=0.0 *P=0.049*** | **U=0.0 *P*=*0.049*** |
| **U Mann-Whitney (*vs*. Tourista/NK200) Significativité** | | | | **U=0.0 *P*=*0.049*** |

**Tableau 11 : Comparaison des prises hydriques moyennes (g/kg poids corporel) des rats entre J2 et J3 (Moyenne ± ESM)**

| **Groupe** | **Témoin/Véhicule** | **Tourista/Véhicule** | **Tourista/NK200** | **Tourista/NK400** |
|---|---|---|---|---|
| **Moyenne ± ESM** | **96.25 ± 3.80** | **60.75 ± 18.30** | **58.39 ± 17.84** | **113.47 ± 17.00** |

**Tableau 12 : Comparaison des prises hydriques moyennes (g/kg poids corporel) des rats entre J3 et J4 (Moyenne ± ESM)**

| **Groupe** | **Témoin/Véhicule** | **Tourista/Véhicule** | **Tourista/NK200** | **Tourista/NK400** |
|---|---|---|---|---|
| **Moyenne ± ESM** | **92.99 ± 3.98** | **83.67 ± 11.64** | **82.26 ± 3.76** | **99.82 ± 13.63** |

### Dosage des cytokines

On définit par « cytokine », une molécule peptidique impliquée dans la régulation des fonctions immunitaires en réponse à une infection bactérienne. Les cytokines les plus connues sont les interleukines (IL), les interférons (IFN), les facteurs de croissance hématopoïétiques (CSF) et les facteurs de nécrose des tumeurs (TNF). On distingue les cytokines pro-inflammatoires et les cytokines anti-inflammatoires.

### Cytokines pro-inflammatoires

### IL1-α et II 1-β

L'IL-1 est une cytokine pro-inflammatoire qui agit au niveau de l'hypothalamus lors d'une infection, afin d'induire la synthèse de prostaglandine, ainsi qu'au niveau du foie pour activer la synthèse de molécules de la phase aiguë de l'inflammation. Les deux formes d'interleukine-1 les plus étudiées sont les IL1-α et II 1-β.

### (i) IL1-α

Le test de Kruskal-Wallis montre qu'il y a eu une différence significative entre les taux sériques moyens de IL1-α des rats des 4 groupes de traitement (P=0,015) (cf. Tableau 13). Les taux sériques moyens de IL1-α des rats du groupe Turista/Véhicule a été significativement plus élevé que ceux ses rats des 2 groupes Témoin/Véhicule et Turista/NK400 (U=1,0 ; P=0,007 et U=3,0 ; P=0,016 respectivement) et il a montré une tendance à être significativement plus élevé que celui des rats du groupe Turista/NK200 (U=6,0 ; P=0,055). En revanche, aucune différence significative n'a été observée entre les taux sériques moyens d'IL1-α des rats des groupes Témoin/Véhicule, Turista/NK200 et Turista/NK400 (cf. Tableau 13).

**Tableau 13 : Comparaison des taux sériques d'IL1-α (pg/ml) des rats 3 heures après induction de la turista (Moyenne ± ESM)**

| **Groupe** | **Témoin/Véhicule** | **Tourista/Véhicule** | **Tourista/NK200** | **Tourista/NK400** |
|---|---|---|---|---|
| **Moyenne ± ESM** | **156.3 ± 40.2** | **922.5 ± 266.3** | **359.2 ± 88.4** | **230.2 ± 65.3** |
| **Kruskal-Wallis H_{(ddl-3)} =10.45; P=0.015 U Mann-Whitney (*vs*. Témoin/Véhicule) Significativité** | | **U=1.0 *P*=*0.007*** | **U=9.0 *NS*** | **U=14.0 *NS*** |
| **U Mann-Whitney (*vs*. Tourista/Véhicule) Significativité** | | | **U=6.0 *T (P*=*0.055)*** | **U=3.0 *P*=*0.016*** |
| **U Mann-Whitney (*vs*. Tourista/NK200) Significativité** | | | | **U=12.0 *NS*** |

### (ii) IL1-β

Le test de Kruskal-Wallis montre qu'il y a eu une différence significative entre les taux sériques moyens d'IL1-β des rats des 4 groupes de traitement (P=0,0004) (cf. Tableau 14). Les taux sériques moyens d'IL1-β des rats du groupe Turista/Véhicule et Turista/NK200 ont été significativement plus élevés que ceux des rats des deux groupes Témoin/Véhicule et Turista/NK400 (U=0,0 et P=0,004 dans tous les cas). Le taux sérique d'IL1-β des rats du groupe Turista/Véhicule a montré une tendance à être significativement plus élevé que celui des rats du groupe Turista/NK200 (U=7,0 et P=0,078). En revanche, aucune différence significative n'a été observée entre les taux sériques moyens d'IL1-β des rats des groupes Témoin/Véhicule et Turista/NK400 (U=13,0 et P=0,42) (cf. Tableau 14).

**Tableau 14 : Comparaison des taux sériques d'IL1-β (pg/ml) des rats 3 heures après induction de la turista (Moyenne ± ESM)**

| **Groupe** | **Témoin/Véhicule** | **Tourista/Véhicule** | **Tourista/NK200** | **Tourista/NK400** |
|---|---|---|---|---|
| **Moyenne ± ESM** | **335.1 ± 145.7** | **61133.6 ± 19565.4** | **25333.4 ± 7369.2** | **481.3 ± 148.0** |
| **Kruskal-Wallis H_{(ddl-3)} =18.25; P=0.0004 U Mann-Whitney (*vs*. Témoin/Véhicule) Significativité** | | **U=0.0 *P*=*0*.*004*** | **U=0.0 *P*=*0.004*** | **U=13.0 *NS*** |
| **U Mann-Whitney (*vs*. Tourista/Véhicule) Significativité** | | | **U=7.0 *T (P*=*0.078)*** | **U=0.0 *P*=*0.004*** |
| **U Mann-Whitney (*vs*. Tourista/NK200) Significativité** | | | | **U=0.0 *P*=*0.004*** |

### (iii) IL-2

Le test de Kruskal-Wallis montre qu'il n'y a pas eu de différences significatives entre les taux sériques moyens d'IL-2 des rats des 4 groupes de traitement mais une tendance à la significativité a été observée (P=0,06) (cf. Tableau 15).

**Tableau 15 : Comparaison des taux sériques d'IL-2 (pg/ml) des rats 3 heures après induction de la turista (Moyenne ± ESM)**

| **Groupe** | **Témoin/Véhicule** | **Tourista/Véhicule** | **Tourista/NK200** | **Tourista/NK400** |
|---|---|---|---|---|
| **Moyenne ± ESM** | 337.2 ± 182.5 | 2152.8 ± 1711.3 | 895.1 ± 514.9 | 279.3 ± 130.5 |

### (iv) IL-6

L'IL-6 est une cytokine pro-inflammatoire qui va agir au niveau du foie lors d'une infection, afin d'activer la synthèse de molécules de la phase aiguë de l'inflammation._Le test de Kruskal-Wallis montre qu'il y a eu une différence significative entre les taux sériques moyens d'IL-6 des rats des 4 groupes de traitement (P=0,0004) (cf. Tableau 16). Les taux sériques moyens d'IL-6 des rats des groupes Turista/Véhicule et Turista/NK200 ont été significativement plus élevés que ceux des rats des 2 groupes Témoin/Véhicule et Turista/NK400 (U =0,0 et P=0,004 dans tous les cas). Le taux sérique moyen d'IL-6 des rats du groupe Turista/NK400 a montré une tendance à être significativement plus élevé que celui des rats du groupe Témoin/Véhicule (U =6,0 et P=0,054). En revanche, aucune différence significative n'a été observée entre les taux sériques moyens d'IL-6 des rats des groupes Turista/Véhicule et Turista/NK200 (U=13,0 et P=0,42) (cf. Tableau 16).

**Tableau 16 : Comparaison des taux sériques d'IL-6 (pg/ml) des rats 3 heures après induction de la turista (Moyenne ± ESM)**

| **Groupe** | **Témoin/Véhicule** | **Tourista/Véhicule** | **Tourista/NK200** | **Tourista/NK200** |
|---|---|---|---|---|
| **Moyenne ± ESM** | 217.6 ± 39.7 | 97915.8 ± 29576.6 | 52013.4 ± 12591.5 | 329.7 ± 49.8 |
| Kruskal-Wallis H_{(dd-3)} =18.42; P=0.0004 U Mann-Whitney (*vs*. Témoin/Véhicule) Sïgnfficativité | | U=0.0 *P=0.004* | U=0.0 *P=0.004* | U=6.0 *T (P=0.054)* |
| U Mann-Whitney (*vs*. Tourista/Véhicule) Significativité | | | U=13.0 *NS* | U=0.0 *P=0.004* |
| U Mann-Whitney (*vs*. Tourista/NK200) Significativité | | | | U=0.0 *P=0.004* |

### (v) GM-CSF

Le test de Kruskal-Wallis montre qu'il y a eu une différence significative entre les taux sériques moyens de GM-CSF des rats des 4 groupes de traitement (P=0,0005) (Tableau 17). Les taux sériques moyens de GM-CSF des rats des groupes Turista/Véhicule et Turista/NK200 ont été significativement plus élevés que ceux des rats des 2 groupes Témoin/Véhicule (U=0,5, P=0,005 et U=0,0, P=0,004, respectivement) et Turista/NK400 (U=1,0, P=0,006 et U=0,0, P=0,004, respectivement). En revanche, aucune différence significative n'a été observée entre les taux sériques moyens de GM-CSF des rats des groupes Témoin/Véhicule et Turista/NK400 (U=8,5 et P=0,12) et entre ceux des rats des groupes Turista/Véhicule et Turista/NK200 (U=13,0 et P=0,41) (Tableau 17).

**Tableau 17 : Comparaison des taux sériques de GM-CSF (pg/ml) des rats 3 heures après induction de la turista (Moyenne ± ESM)**

| **Groupe** | **Témoin/Véhicule** | **Tourista/Véhicule** | **Témoin/NK200** | **Tourista/NK400** |
|---|---|---|---|---|
| **Moyenne ± ESM** | 14.0 ± 3.3 | 65.1 ± 17.7 | 48.1 ± 1.7 | 22.5 ± 4.0 |
| Kruskal-Wallis H_{(ddl-3)}=17.54; P=0.0005 U Mann-Whitney (*vs*. Témoin/Véhicule) Significativaté | | U=0.5 *P*=*0.005* | U=0.0 *P=0.004* | U=8.5 *NS* |
| U Mann-Whitney (*vs*. Tourista/Véhicule) Significativité | | | U=13.0 *NS* | U=1.0 *P=0.006* |
| U Mann-Whitney (*vs*. Tourista/NK200) Significativité | | | | U=0.0 *P=0.004* |

### (vi) IFN-γ

Le test de Kruskal-Wallis montre qu'il y a eu une différence significative entre les taux sériques moyens d'IFN-γ des rats des 4 groupes de traitement (P=0,0005) (cf. Tableau 18). Les taux sériques moyens d'IFN-γ des rats des groupes Turista/Véhicule et Turista/NK200 ont été significativement plus élevés que ceux des rats des 2 groupes Témoin/Véhicule et Turista/NK400 (U =0,0 et P=0,004 dans tous les cas). En revanche, aucune différence significative n'a été observée entre les taux sériques moyens d'IFN-γ des rats des groupes Témoin/Véhicule et Turista/NK400 (U=13,0 et P=0,42) et entre ceux des rats des groupes Turista/Véhicule et Turista/NK200 (U=12,0 et P=0,34) (cf. Tableau 18).

**Tableau 18 : Comparaison des taux sériques d'IFN-γ (pg/ml) des rats 3 heures après induction de la turista (Moyenne ± ESM)**

| **Groupe** | **Témoin/Véhicule** | **Tourista/Véhicule** | **Tourista/NK200** | **Tourista/NK400** |
|---|---|---|---|---|
| **Moyenne ± ESM** | 426.5 ± 55.2 | 23921.1 ± 5491.9 | 16557.2 ± 5976.1 | 432.9 ± 123.0 |
| Kruskal-Wallis H_{(ddl-3)}=17.69; P=0.0005 U Mann-Whitney (*vs*. Témoin/Véhicule) Significativité | | U=0.0 *P=0.004* | U=0.0 *P=0.004* | U=13.0 *NS* |
| U Mann-Whitney (*vs*. Tourista/Véhicule) Significativité | | | U=12.0 *NS* | U=0.0 *P=0.004* |
| U Mann-Whitney (*vs*. Tourista/NK200) Significativité | | | | U=0.0 *P=0.004* |

### (vii) TFN-α

Le TNF-α est une cytokine pro-inflammatoires agissant au niveau du foie lors d'une infection en induisant la synthèse de molécules de la phase aiguë de l'inflammation.

Le test de Kruskal-Wallis montre qu'il y a eu une différence significative entre les taux sériques moyens de TNF-α des rats des 4 groupes de traitement (P=0,0002) (cf. Tableau 19). Les taux sériques moyens de TNF-α des rats des groupes Turista/Véhicule, Turista/NK200 et Turista/NK400 ont été significativement plus élevés que ceux des rats du groupe Témoin/Véhicule (U=0,0 ; P=0,004 dans tous les cas). Les taux sériques moyens de TNF-α des rats des groupes Turista/Véhicule et Turista/NK200 ont été significativement plus élevés que celui des rats du groupe Turista/NK400 (U=0,0 ; P=0,004 dans les deux cas). En revanche, aucune différence significative n'a été observée entre les taux sériques moyens de TNF-α des rats des groupes Turista/Véhicule et Turista/NK200 (U=17,0 et P=0,87) (cf. Tableau 19).

**Tableau 19 : Comparaison des taux sériques de TNF-α (pg/ml) des rats 3 heures après induction de la turista (Moyenne ± ESM)**

| **Groupe** | **Témoin/Véhicule** | **Tourista/Véhicule** | **Tourista/NK200** | **Tourista/NK400** |
|---|---|---|---|---|
| **Moyenne ± ESM** | 81.1 ± 4.5 | 20768.0 ± 10998.7 | 12064.9 ± 5546.5 | 260.1 ± 12.5 |
| Kruskal-Wallis H_{(ddl-3)}=19.45; P=0.0002 U Mann-Whitney (*vs*. Témoin/Véhicule) Significativité | | U=0.0 *P=0.004* | U=0.0 *P=0.004* | U=0.0 *P=0.004* |
| U Mann-Whitney (*vs*. Tourista/Véhicule) Signiticativïté | | | U=17.0 *NS* | U=0.0 *P=0.004* |
| U Mann-Whitney (*vs*. Tourista/NK200) Significativité | | | | U=0.0 *P=0.004* |

### Cytokines anti-inflammatoires

### (viii) IL-4

L'IL-4 est une cytokine produite par la cellule NKT (natural killer)T. Le test de Kruskal-Wallis montre qu'il y a eu une différence significative entre les taux sériques moyens d'IL-4 des rats des 4 groupes de traitement (P=0,001) (cf. Tableau 20). Les taux sériques moyens d'IL-4 des rats du groupe Turista/Véhicule et Turista/NK200 et Turista/NK400 ont été significativement plus élevés que ceux des rats des deux groupes Témoin/Véhicule (U=0,0 et P=0,004 ; U=0,0 et P=0,004 ; U=5,0 et P=0,037 respectivement). Le taux sérique moyens d'IL-4 des rats du groupe Turista/Véhicule et Turista/NK200 ont été significativement plus élevés que celui des rats du groupe Turista/NK400 (U=3,0 et P=0,016 dans les 2 cas). En revanche, aucune différence significative n'a été observée entre les taux sériques moyens d'IL-4 des rats des groupes Témoin/Véhicule et Turista/NK200 (U=18,0 et P > 0,99) (cf. Tableau 20).

**Tableau 20 : Comparaison des taux sériques d'IL-4 (pg/ml) des rats 3 heures après induction de la turista (Moyenne ± ESM)**

| **Groupe** | **Témoin/Véhieule** | **Tourista/Véhicule** | **Tourista/NK200** | **Tourista/NK400** |
|---|---|---|---|---|
| **Moyenne ± ESM** | 3.0 ± 0.6 | 34.4 ± 13.1 | 29.5 ± 6.8 | 7.5 ± 2.5 |
| **Kruskal-Wallis** H_{(ddl-3)}=1624; P=0.001 U Mann-Whitney (*vs*. Témoin/Véhicule) Significativité | | U=0.0 *P=0.004* | U=0.0 *P=0.004* | U=5.0 *P=0.037* |
| U Mann-Whitney (*vs*. Touriste/Véhicule) Significativité | | | U=18.0 *NS* | U=3.0 *P=0.016* |
| U Mann-Whitney (*vs*. Tourista/NK200) Significativité | | | | U=3.0 *P=0.016* |

### (ix) IL-10

L'IL-10 est une cytokine anti-inflammatoire, jouant un rôle de régulation de la réaction inflammatoire, permettant ainsi qu'elle ne devienne pas exagérée et donc pathologique. Le test de Kruskal-Wallis montre qu'il y a eu une différence significative entre les taux sériques moyens d'IL-10 des rats des 4 groupes de traitement (P=0,0002) (cf. Tableau 21). Les taux sériques moyens d'IL-10 des rats du groupe Turista/Véhicule et Turista/NK200 et Turista/NK400 ont été significativement plus élevés que ceux des rats des deux groupes Témoin/Véhicule (U=0,0 et P=0,004 ; U=0,0 et P=0,004 ; U=2,0 et P=0,010 respectivement). Le taux sérique moyens d'IL-10 des rats du groupe Turista/Véhicule et Turista/NK200 ont été significativement plus élevés que celui des rats du groupe Turista/NK400 (U=0,0 et P=0,004 ; U=1,0 et P=0,007 respectivement). En revanche, aucune différence significative n'a été observée entre les taux sériques moyens d'IL-4 des rats des groupes Témoin/Véhicule et Turista/NK200 (U=8,0 et P=0,11) (cf. Tableau 21). Tableau 21 : Comparaison des taux sériques d'IL-10 (pg/ml) des rats 3 heures après

**induction de la turista (Moyenne ± ESM)**

| **Groupe** | **Témoin/Véhicule** | **Tourista/Véhicule** | **Tourista/NK200** | **Tourista/NK400** |
|---|---|---|---|---|
| **Moyenne ± ESM** | 224.3 ± 34.2 | 3523.7 ± 449.2 | 2293.5 ± 345.7 | 559.3 ± 133.6 |
| Kruskal-Wallis H_{(ddl-3)}=1.9.35; P=0.0002 U Mann-Whitney (*vs*. Témoin/Véhicule) Significativité | | U=0.0 *P=0.004* | U=0.0 *P=0.004* | U=2.0 *P=0.010* |
| U Mann-Whitney (*vs*. Tourista/Véhicule) Significativité | | | U=8.0 *NS* | U=0.0 *P=0.004* |
| U Mann-Whitney (*vs*. Tourista/NK200) Significativité | | | | U=1.0 *P=0.007* |

### Consistance des fèces

A J1 après l'induction de la turista, 5 rats sur 6 du groupe Turista/Véhicule ont présenté une diarrhée avec émission de fèces sous formes liquide, le 6^{ème} rat ayant à la fois une diarrhée avec émission de fèces sous forme liquide et émission de fèces molles. En ce qui concerne le groupe de traitement Turista/NK200, 3 rats sur 6 ont présenté une diarrhée avec émission de fèces sous forme liquide, les 3 autres rats ont présenté une diarrhée avec émission de fèces molles. En ce qui concerne le groupe de traitement Turista/NK400, les 6 rats ont présenté des fèces molles. Enfin, les 6 rats du groupe Témoin/Véhicule ont présenté des fèces dures.

A J2, soit 24 heures après l'induction de la turista, 1 rat sur 6 du groupe Turista/Véhicule a présenté une diarrhée avec émission de fèces sous forme liquide, les 5 autres ayant des fèces molles tout comme les rats du groupe Turista/NK200. En ce qui concerne le groupe Turista/NK400, les 6 rats ont présentés des fèces dures et molles. Enfin, les 6 rats du groupe Témoin/Véhicule ont présenté des fèces dures.

A J3, soit 48 heures après l'induction de la turista, les 6 rats du groupe Turista/Véhicule ont présenté des fèces molles. Les 6 rats du groupe Turista/NK200 ont présenté des fèces dures et molles. Enfin, les 6 rats du groupe Turista/NK400 ont présenté des fèces dures tout comme les 6 rats du groupe Témoin/Véhicule.

A J4, soit 72 heures après l'induction de la turista, les 6 rats du groupe Turista/Véhicule ont présenté des fèces dures et molles. Les 6 rats du groupe Turista/NK200 ont présenté des fèces dures comme d'ailleurs les 6 rats des groupes Turista/NK400 et Témoin/Véhicule.

Ainsi, le Nekka-Rich administré par voie orale en traitement curatif à la dose de 400 mg/kg chez des rats mâles Wistar soumis à l'induction de turista par une administration orale de bactéries Escherichia coli, a un effet significatif et bénéfique sur le traitement de cette pathologie. En effet, l'étude a permis de limiter de manière significative les variations de poids, de température corporelles, de prises alimentaire et hydrique des animaux, d'empêcher la production de diarrhée et également de limiter significativement la production de cytokines pro- et anti-inflammatoires.

Par extrapolation de ces résultats sur l'homme ou sur d'autres animaux, le Nekka-Rich peut être utilisé pour le traitement préventif de pathologies intestinales provoqués par la bactérie Escherichia coli entérotoxinogène (ECET) dans les cas suivants :
- la turista humaine, par prise de dose préventive journalière pendant toute la période de risque de contamination, c'est à dire lors de la durée du voyage dans un pays à risque ;
- les diarrhées colibacillaires à souche ECET du veau, du chevreau, de l'agneau et des carnivores domestiques, tels que les chiens et les chats.

En effet, une dose de 400 mg/kg chez le rat équivaut à une dose de 64,5 mg/kg chez un homme de 60 kg. Ainsi, pour le traitement préventif de la tourista, les doses de principe actif (Nekka-Rich) peuvent être les suivantes :
- 1 g pour 15 kg de poids vif (PV) quelque soit l'âge de la personne le matin et le soir, par voie orale, pendant toute la période d'exposition aux pathogènes de la turista ;
- pour un enfant de plus de 30 kg : 2,5 g de Nekka-Rich le matin et le soir par voie orale pendant toute la période d'exposition aux pathogènes de la turista.

En ce qui concerne les animaux, un exemple de doses journalières de Nekka-Rich à utiliser pour le traitement prévention de colibacille à souche ECET est présenté dans le tableau 22 ci-après.

**Tableau 22 : Dose (en grammes) de Nekka-Rich pour la prévention de colibacille à souche ECET pour l'agneau, le chevreau, le veau et les carnivores domestiques et chats).**

| | **Dose le matin (g)** | **Dose le soir (g)** |
|---|---|---|
| **Agneau ou chevreau** | | |
| ≤ 10 kg | 1,2 | 1,2 |
| > 10 kg | 2 | 2 |

| **Veau** | | |
|---|---|---|
| ≤ 40 kg | 5 | 5 |
| 40 < X ≤ 60 kg | 7,5 | 7,5 |
| > 60 kg | 10 | 10 |

| **Carnivores domestiques** | | |
|---|---|---|
| ≤ 5 kg | 0,6 | 0,6 |
| 5 < X ≤ 15kg | 1,7 | 1,7 |
| 15 < X ≤ 30 kg | 3,5 | 3,5 |
| > 30 kg | 5 | 5 |

## Revendications

1. Composition à base d'extraits d'écorces de bois de chêne (*Castanopsis cuspidata* et/ou *Quercus acuta*) comprenant à titre de principe actif le Nekka-Rich pour son utilisation pour le traitement préventif et/ou curatif de pathologies intestinales provoquées par la bactérie Escherichia coli entérotoxinogène (ECET) chez l'homme ou chez les animaux mammifères.

2. Composition pour son utilisation selon la revendication 1, **caractérisée en ce qu'**elle comprend à titre de principe actif du Nekka-Rich en association ou en mélange avec au moins un excipient ou véhicule inerte et non toxique.

3. Composition pour son utilisation selon la revendication 1 ou 2, **caractérisée en ce que** la composition comprend en outre au moins un additif, de préférence sélectionné dans le groupe formé par :
- les vitamines A, E, D, C synthétiques ou naturelles ;
- les oligo-éléments, et notamment le fer, l'iode, le sélénium, le zinc, le cuivre, le cobalt, le manganèse, sous leur forme minérale ou organique ;
- des extraits de *macleaya cordata* ;
- les argiles, et notamment la montmorillonite, la bentonite, la smectite ;
- les pro-biotiques tels que les lactobacillus ou les saccharomyces ;
- les anti-diarrhéiques.
- les antispasmodiques ;
- les antiseptiques intestinaux, et notamment des nitrofuranes ou des quinoléines.

4. Composition pour son utilisation selon l'une quelconque des revendications 1 à 3, **caractérisée en ce que** la composition se présente sous forme solide, notamment sous la forme d'un comprimé compressé, d'une gélule, d'une poudre orale, d'une poudre en sachet ; ou sous forme liquide, notamment sous la forme d'un gel, d'une émulsion, d'une mousse, d'une pâte ou d'une suspension.

5. Composition pour son utilisation selon la revendication 4, **caractérisée en ce que** la composition sous forme solide destinée à l'homme comprend entre 10 g à 100 g de Nekka-Rich pour 100 g de préparation, de préférence 30 g à 100 g de Nekka-Rich pour 100 g de préparation, et encore plus préférentiellement 60 g à 100 g de Nekka-Rich pour 100 g de préparation.

6. Composition pour son utilisation selon la revendication 4, **caractérisée en ce que** la composition sous forme solide destinée aux animaux comprend entre 10 g à 100 g de Nekka-Rich pour 100 g de préparation, de préférence 30 g à 100 g Nekka-Rich pour 100 g de préparation, et encore plus préférentiellement 45 g à 100 g de Nekka-Rich pour 100 g de préparation.

7. Composition pour son utilisation selon la revendication 4, **caractérisée en ce que** la composition sous forme liquide destinée à l'homme comprend entre 20 g à 600 g de Nekka-Rich pour 1 litre de préparation, de préférence 100 g à 450 g de Nekka-Rich pour 1 litre de préparation, et encore plus préférentiellement 250 g à 350 g de Nekka-Rich pour 1 litre de préparation.

8. Composition pour son utilisation selon la revendication 4, **caractérisée en ce que** la composition sous forme liquide destinée aux animaux comprend entre 20 g à 600 g de Nekka-Rich pour 1 litre de préparation, de préférence 100 g à 450 g Nekka-Rich pour 1 litre de préparation, et encore plus préférentiellement 250 g à 350 g de Nekka-Rich pour 1 litre de préparation.

9. Composition pour son utilisation selon l'une quelconque des revendications 1 à 8 pour une utilisation comme complément alimentaire par administration par voie orale chez l'homme, à raison de deux prises quotidiennes comprises entre 0,8 g et 1,2 g de Nekka-Rich par 15 kg de poids vif, et comprises entre 2,2 g et 2,7 g de Nekka-Rich pour un enfant de plus de 30 kg.

10. Composition pour son utilisation selon l'une quelconque des revendications 1 à 8 pour une utilisation comme aliment complémentaire par administration par voie orale chez le jeune mammifère, à raison de deux prises quotidiennes, l'une le matin et l'autre le soir, et notamment :
(i) comprises entre 1,0 et 1,5 g chacune de Nekka-Rich chez l'agneau ou le chevreau de 10 kg ou moins de 10 kg ;
(ii) comprises entre 1,8 et 2,2 g chacune de Nekka-Rich chez l'agneau ou le chevreau de plus de 10 kg ;
(iii) comprises entre 4 et 6 g chacune de Nekka-Rich pour un veau de 40 kg ou de moins de 40 kg ;
(iv) comprises entre 7 et 8 g chacune de Nekka-Rich pour un veau entre 40 kg et 60 kg ou de 60 kg ;
(v) comprises entre 9 et 11 g chacune de Nekka-Rich pour un veau de plus de 60 kg ;
(vi) comprises entre 0,2 et 1 g chacune de Nekka-Rich pour un carnivore domestique de 5 kg ou moins de 5 kg ;
(vii) comprises entre 1,5 et 2 g chacune de Nekka-Rich pour un carnivore domestique entre 5 kg et 15 kg ou de 15 kg ;
(viii) comprises entre 3 et 4 g chacune de Nekka-Rich pour un carnivore domestique de entre 15 kg et 30 kg ou de 30 kg ;
(ix) comprises entre 4,5 et 6 g chacune de Nekka-Rich pour un carnivore domestique de plus de 30 kg.

11. Complément alimentaire ou aliment complémentaire comprenant une composition pour son utilisation selon l'une quelconque des revendications 1 à 8.

## Patentansprüche

1. Zusammensetzung auf der Basis von Rindenextrakten von Eichenholz (*Castanopsis cuspidata* und/oder *Quercus acuta*)*,* die als Wirkstoff Nekka-Rich umfasst, zu deren Verwendung zur Präventativ- und/oder Heilbehandlung von Darmkrankheiten, die von dem Bakterium enterotoxinogenes Escherichia coli (ETEC) verursacht werden, beim Menschen oder bei Säugetieren.

2. Zusammensetzung zu deren Verwendung nach Anspruch 1, **dadurch gekennzeichnet, dass** sie als Wirkstoff Nekka-Rich in Verbindung oder im Gemisch mit mindestens einem inerten und nichttoxischen Hilfsstoff oder Trägerstoff umfasst

3. Zusammensetzung zu deren Verwendung nach Anspruch 1 oder 2, **dadurch gekennzeichnet, dass** die Zusammensetzung außerdem mindestens ein Additiv umfasst, das vorzugsweise aus der Gruppe ausgewählt ist, die von folgenden gebildet wird:
- den Vitaminen A, E, D, C, synthetisch oder natürlich;
- den Oligoelementen und insbesondere Eisen, Iod, Selen, Zink, Kupfer, Kobalt, Mangan, in ihrer mineralischen oder organischen Form;
Extrakten von *Macleaya cordata;*
- den Tonen und insbesondere dem Montmorillonit, dem Bentonit, dem Smektit;
- den Probiotika, wie den Lactobacillus und den Saccharomyces;
- den Antidiarrhoika;
- den Spasmolytika;
- den Darm-Antiseptika und insbesondere Nitrofurane oder Chinoleine.

4. Zusammensetzung zu deren Verwendung nach einem der Ansprüche 1 bis 3, **dadurch gekennzeichnet, dass** die Zusammensetzung in fester Form, insbesondere in der Form einer komprimierten Tablette, einer Kapsel, eines Pulvers zur oralen Anwendung, eines Pulvers in einem Sachet; oder in flüssiger Form, insbesondere in der Form eines Gels, einer Emulsion, eines Schaums, einer Paste oder einer Suspension, vorliegt

5. Zusammensetzung zu deren Verwendung nach Anspruch 4, dadurch gekenntzeichnet, dass die Zusammensetzung in fester Form, die für den Menschen vorgesehen ist, zwischen 10 g und 100 g Nekka-Rich pro 100 g Präparat, vorzugsweise 30 g bis 100 g Nekka-Rich pro 100 g Präparat und noch mehr bevorzugt 60 g bis 100 g Nekka-Rich pro 100 g Präparat umfasst.

6. Zusammensetzung zu deren Verwendung nach Anspruch 4, **dadurch gekennzeichnet, dass** die Zusammensetzung in fester Form, die für Tiere vorgesehen ist, zwischen 10 g und 100 g Nekka-Rich pro 100 g Präparat, vorzugsweise 30 g bis 100 g Nekka-Rich pro 100 g Präparat und noch mehr bevorzugt 45 g bis 100 g Nekka-Rich pro 100 g Präparat umfasst

7. Zusammensetzung zu deren Verwendung nach Anspruch 4, **dadurch gekennzeichnet, dass** die Zusammensetzung in flüssiger Form, die für den Menschen vorgesehen ist, zwischen 20 g und 600 g Nekka-Rich pro 1 Liter Präparat, vorzugsweise 100 g bis 450 g Nekka-Rich pro 1 Liter Präparat und noch mehr bevorzugt 250 g bis 350 g Nekka-Rich pro 1 Liter Präparat umfasst

8. Zusammensetzung zu deren Verwendung nach Anspruch 4, **dadurch gekennzeichnet, dass** die Zusammensetzung in flüssiger Form, die für Tiere vorgesehen ist, zwischen 20 g und 600 g Nekka-Rich pro 1 Liter Präparat, vorzugsweise 100 g bis 450 g Nekka-Rich pro 1 Liter Präparat und noch mehr bevorzugt 250 g bis 350 g Nekka-Rich pro 1 Liter Präparat umfasst.

9. Zusammensetzung zu deren Verwendung nach einem der Ansprüche 1 bis 8 für eine Verwendung als Nahrungsergänzungsmittel zur oralen Verabreichung an den Menschen zu zwei täglichen Einnahmen, die zwischen 0,8 g und 1,2 g Nekka-Rich pro 15 kg Lebendgewicht liegen und zwischen 2,2 g und 2,7 g Nekka-Rich für ein Kind von mehr als 30 kg liegen

10. Zusammensetzung zu deren Verwendung nach einem der Ansprüche 1 bis 8 für eine Verwendung als Ergänzungsfutter zur oralen Verabreichung an ein junges Säugetier zu zwei täglichen Einnahmen, eine morgens und die andere abends, und die insbesondere:
(i) zwischen jeweils 1,0 und 1,5 g Nekka-Rich beim Lamm oder Kitz von 10 kg oder weniger als 10 kg liegen;
(ii) zwischen jeweils 1,8 und 2,2 g Nekka-Rich beim Lamm oder Kitz von mehr als 10 kg liegen;
(iii) zwischen jeweils 4 und 6 g Nekka-Rich für ein Kalb von 40 kg oder weniger als 40 kg liegen;
(iv) zwischen jeweils 7 und 8 g Nekka-Rich für ein Kalb zwischen 40 kg und 60 kg oder von 60 kg liegen;
(v) zwischen jeweils 9 und 11 g Nekka-Rich für ein Kalb von mehr als 60 kg liegen;
(vi) zwischen jeweils 0,2 und 1 g Nekka-Rich für ein fleischfressendes Haustier von 5 kg oder weniger als 5 kg liegen;
(vii) zwischen jeweils 1,5 und 2 g Nekka-Rich für ein fleischfressendes Haustier zwischen 5 kg und 15 kg oder von 15 kg liegen;
(viii) zwischen jeweils 3 und 4 g Nekka-Rich für ein fleischfressendes Haustier zwischen 15 kg und 30 kg oder von 30 kg liegen;
(ix) zwischen jeweils 4,5 und 6 g Nekka-Rich für ein fleischfressendes Haustier von mehr als 30 kg liegen.

11. Nahrungsergänzungsmittel oder Ergänzungsfutter, das eine Zusammensetzung zu deren Verwendung nach einem der Ansprüche 1 bis 8 umfasst

## Claims

1. Composition based on oak (*Castanopsis cuspidata* and/or *Quercus acuta*) wood bark extracts comprising, by way of active ingredient, Nekka-Rich for use for the preventive and/or curative treatment of intestinal diseases caused by enterotoxigenic Escherichia coli (ETEC) in humans or in mammalian animals.

2. Composition for use according to claim 1, **characterised in that** it comprises, by way of active ingredient, Nekka-Rich in association or mixed with at least one inert and non-toxic excipient or vehicle.

3. Composition for use according to claim 1 or 2, **characterised in that** the composition further comprises at least one additive, preferably selected in the group formed by:
synthetic or natural vitamins A, E, D, C;
- trace elements, and notably iron, selenium, zinc, copper, cobalt, manganese, in the mineral or organic form thereof;
- *macleaya cordata* extracts;
- clays, and notably montmorillonite, bentonite, smectite; probiotics such as lactobacillus or saccharomyces; antidiarrhoeals;
- antispasmodics;
intestinal antiseptics and notably nitrofurans or quinolines

4. Composition for use according to any one of claims 1 to 3, **characterised in that** the composition is presented in solid form, notably in the form of a compressed tablet, a hard capsule, an oral powder, a powder in a sachet; or in liquid form, notably in the form of a gel, an emulsion, a foam, a paste or a suspension.

5. Composition for use according to claim 4 **characterised in that** the composition in solid form intended for humans comprises between 10 g and 100 g of Nekka-Rich per 100 g of preparation, preferably 30 g to 100 g of Nekka-Rich per 100 g of preparation, and more preferentially 60 g to 100 g of Nekka-Rich per 100 g of preparation

6. Composition for use according to claim 4, **characterised in that** the composition in solid form intended for animals comprises between 10 g and 100 g of Nekka-Rich per 100 g of preparation, preferably 30 g to 100 g of Nekka-Rich per 100 g of preparation, and more preferentially 45 g to 100 g of Nekka-Rich per 100 g of preparation.

7. Composition for use according to claim 4 **characterised in that** the composition in liquid form intended for humans comprises between 20 g and 600 g of Nekka-Rich per 1 litre of preparation, preferably 100 g to 450 g of Nekka-Rich per 1 litre of preparation, and more preferentially 250 g to 250 g of Nekka-Rich per 1 litre of preparation

8. Composition for use according to claim 4 **characterised in that** the composition in liquid form intended for animals comprises between 20 g and 600 g of Nekka-Rich per 1 litre of preparation, preferably 100 g to 450 g of Nekka-Rich per 1 litre g of preparation, and more preferentially 250 g to 350 g of Nekka-Rich per 1 litre of preparation.

9. Composition for use according to any one of claims 1 to 8 for use as a dietary supplement by oral administration to humans, at a rate of two daily doses between 0.8 g and 1.2 g of Nekka-Rich per 15 kg of live weight, and between 2.2 g and 2.7 g of Nekka--Rich for a child of over 30 kg

10. Composition for use according to any one of claims 1 to 8 for use as a supplementary feed by oral administration in young mammals, at a rate of two daily doses, one in the morning and the other in the evening, and notably:
(i) between 1 0 and 1.5 g each of Nekka-Rich in lambs or kids of 10 kg or less than 10 kg;
(ii) between 1.8 and 2.2 g each of Nekka-Rich in lambs or kids of more than 10 kg;
(iii) between 4 and 6 g each of Nekka-Rich for a calf of 40 kg or less than 40 kg;
(iv) between 7 and 8 g each of Nekka-Rich for a calf of 40 kg and 60 kg or of 60 kg;
(v) between 9 and 11 g each of Nekka-Rich for a calf of more than 60 kg;
(vi) between 0.2 and 1 g Nekka-Rich for a domestic carnivore of 5 kg or than 5 kg;
(vii) between 1.5 and 2 g each of Nekka-Rich for a domestic carnivore between 5 kg and 15 kg or of 150 kg;
(viii) between 3 and 4 g each of Nekka-Rich for a domestic carnivore between 15 kg and 30 kg or of 30 kg;
(ix) between 4.5 and 6 g each of Nekka-Rich for a domestic carnivore of more than 30 kg.

11. Dietary supplement or supplementary feed comprising a composition for use according to any one of claims 1 to 8
